# EUROPÄISCHE PATENTSCHRIFT

(11) **EP 1 132 041 B1**
(45) Veröffentlichungstag und Bekanntmachung des Hinweises auf die Patenterteilung: **09.06.2004**
(21) Anmeldenummer: 01105299.0
(22) Anmeldetag: 05.03.2001
(51) Int. Cl.: A61B 1/005, A61B 1/018

(54) **Endoskopschaft**
Endoscope shaft
Tige endoscopique

(30) Priorität: 06.03.2000 DE 10010931
(43) Veröffentlichungstag der Anmeldung: 12.09.2001
(73) Patentinhaber: STM Medizintechnik Starnberg GmbH, 86947 Schwabhausen/Weil (DE)
(72) Erfinder: Pauker, Fritz, 86316 Friedberg (DE)
(74) Vertreter: Leson, Thomas Johannes Alois, Dipl.-Ing.

(56) Entgegenhaltungen:
- DE-A- 4 133 605
- DE-A- 4 242 291
- US-A- 4 580 551
- US-A- 5 337 732
- PATENT ABSTRACTS OF JAPAN vol. 1999, no. 10, 31. August 1999 (1999-08-31) & JP 11 137510 A (OLYMPUS OPTICAL CO LTD), 25. Mai 1999 (1999-05-25)

## Beschreibung

Die vorliegende Erfindung betrifft einen Endoskopschaft gemäß dem Oberbegriff des Patentanspruchs 1.

Endoskope sind Geräte, insbesondere zur Exploration von Hohlräumen oder röhrenartigen Kanälen des Körpers beispielsweise für medizinische Zwecke. Derartige Endoskope werden vorzugsweise zur Exploration der Speiseröhre, des Magens, des Zwölffingerdarms vom Magen aus, des Darms vom Anus aus, der Harnröhre, der Blase und der Harnleiter verwendet. Zumeist sind Endoskope an ihrem Vorderende mit einer Beleuchtungseinrichtung und mit einer Optik zur visuellen Erfassung des davor liegenden Bereichs des Körperhohlraums oder Körperkanals ausgerüstet.

Endoskope weisen ferner in der Regel einen sog. Arbeitskanal auf, durch den diverse Arbeitsinstrumente eingeführt und bedient werden können, z. B. Zangen zur Entnahme von Gewebeproben, Biopsienadeln, beheizbare Schneiddrähte, kleinere Scheren oder dergleichen. Schließlich sind in der Regel Funktionskanäle beispielsweise ein Fluidkanal für Spülflüssigkeit und Bedienungsdrähte zum Abwinkeln des Endoskopvorderendes in mehrere Richtungen vorhanden. Insgesamt hat das Endoskop, abgesehen von seinem hinteren Bedienungsende und einem Anschlußstrang, eine langgestreckte, biegsamstabförmige Gestalt. Übliche Außendurchmesser liegen etwa im Bereich von 9 bis 15 mm, am vorderen Kopf etwas größer.

Bisher werden Endoskope dadurch in den Körper eingeführt, dass der Arzt von dem aus dem Körper herausragenden Teil des Endoskops her das drucksteife Endoskop bzw. den drucksteifen Endoskopschaft in den Körper hinein schiebt. Diese Art des Einführens des Endoskops ist besonders mühsam, schwierig und zeitraubend insbesondere beim Koloskop, da im letzteren Fall der Darm Abbiegungen und häufig Engstellen aufweist. Demzufolge gehören koloskopische Untersuchungen bisher zu den aufwendigen und für den Patienten unangenehmen Untersuchungen und kommen deshalb für eine breite Anwendung kaum in Betracht. Das Umgehen mit einem Koloskop erfordert zudem einen hiermit erfahrenen Arzt.

Des weiteren hat sich aus dem Stand der Technik gezeigt, dass Endoskope der bekannten Bauart aufgrund der von ihnen geforderten Steifigkeit für ein Einschieben in den zu untersuchenden Patientenhohlraum, bei gleichzeitiger Biegefähigkeit äußerst aufwendige und damit kostenintensive Konstruktionen darstellen. Diese Konstruktionen sind derart teuer, dass sie immer wieder verwendet müssen. Es ist daher notwendig, nach jeder Untersuchung aufwendige Sterilisationsmaßnahmen durchzuführen, wobei letztlich auch eine Gefahr einer Beschädigung des Endoskopschafts besteht, insbesondere dann, wenn ungeschultes Personal derartige Sterilisationsvorgänge durchführt.

Im Oberbegriff des Patentanspruchs 1 wird indessen von einem Endoskopschaft ausgegangen, wir er in der US-A-4 580 551 gezeigt ist. Der aus dieser Druckschrift bekannte Endoskopschaft hat ein vorderes bzw. distales abwinkelbares Endstück, welches einstückig mit einem geschlossenen Schlauchkörper ausgebildet ist. Der Schlauchkörper besteht wiederum aus einem extrudierten Kunststoffmaterial, welches an seinem distalen, abkrümmbaren Ende mit einem mantelartigen Bauteil umgeben ist.

Angesichts dieses Stands der Technik besteht die Aufgabe der Erfindung darin, einen Endoskopschaft zu schaffen, der wesentlich kostengünstiger herstellbar ist, und bei welchem die Gefahr eines Beschädigens beispielsweise im Rahmen von Sterilisationsvorgängen beseitigt ist.

Diese Erfindung wird durch einen Endoskopschaft mit Merkmalen des Anspruchs 1 gelöst.

Die Erfindung besteht demnach darin, dass der Schlauchkörper an seiner Mantelfläche eine Anzahl von Längsausnehmungen oder Nuten hat, die zusammen mit der Siliconhülle, welche den Schlauchkörper umgibt, Funktionskanäle ausbilden. Hierdurch wird die Herstellung des Endoskopschafts mit einer Anzahl von Funktionskanälen vereinfacht. Darüber hinaus wird der Vorteil erzielt, dass durch die Materialausdünnung insbesondere an der Außenseite des Schlauchkörpers eine höhere Flexibilität des Schafts erzielt wird.

Die Erfindung geht dabei grundsätzlich von der folgenden Überlegung aus:

Aus dem Stand der Technik, insbesondere gemäß der DE 42 42 291 A1 ist ein Endoskop dieser Gattung bekannt. Dieses Endoskop besteht im wesentlichen aus einem Endoskopkopf, oder distalen Ende, an das sich ein Endoskopschaft aus einem wie eingangs beschriebenen flexiblen biegsamen jedoch schubsteifen Rohrkörper anschließt und einer Bedienungseinrichtung am unteren Ende des Endoskopschafts. Die Bedienungseinrichtung hat eine Anzahl von drehbar am Endoskopschaft gelagerten Betätigungsrädern, die über Bedienungsdrähte oder Bautenzüge, welche innerhalb des Endoskopschafts verschieblich verlegt sind, mit dem distalen Ende wirkverbunden sind.

Für ein Einführen dieses Endoskopschafts beispielsweise in den Darm eines zu behandelnden Patienten verwendet dieser Stand der Technik eine Art Doppelstülpschlauch-System, wie es nachfolgend kurz beschrieben wird:

Das aus dieser Druckschrift bekannte Doppelstülpschlauchsystem sieht vor, den Endoskopschaft in einem beidseitig gestülpten Schlauch gleitend zu führen, der wiederum durch eine Antriebseinrichtung fortbewegbar ist, des auf den hierbei sich ausbildenden inneren Schlauchabschnitt des Stülpschlauchs einwirkt. Die Antriebseinrichtung hat zumindest ein kontinuierlich antreibendes Vorschubmittel, beispielsweise eine Anzahl von Antriebsrädern, welches radial auf den inneren Schlauchabschnitt pressbar ist, um diesen in Axialrichtung des Schafts im wesentlichen kontinuierlich zu bewegen. Dies hat den Vorteil, dass der kontinuierliche Vortrieb des Stülpschlauchsystems exakt steuerbar und damit beispielsweise das distale Ende des Endoskops ortsgenau führbar ist.

Hierbei ist es vorgesehen, dass die Anpresskraft des Vorschubmittels auf den inneren Schlauchabschnitt so gewählt ist, dass der Schaft zumindest im Bereich des Vorschubmittels im direkten Reibkontakt mit dem inneren Schlauchabschnitt ist. Das Vorschubmittel wird von einem oder mehreren Reibrädern gebildet, wie vorstehend bereits angedeutet wurde, die gegen den inneren Schlauchabschnitt mit einer vorbestimmten oder einstellbaren Anpresskraft vorspannbar sind, so dass zum einen ein kontinuierlicher und zum anderen ein möglichst schlupffreier Vorschub des Endoskopschafts in den Hohlraum eines Patienten gewährleistet wird. Dabei bildet der Endoskopschaft selbst das Widerlager der Reibräder.

Des weiteren hat die Antriebseinrichtung eine Vorrichtung zur Synchronisation der Schaftbewegung mit der Stülpschlauchbewegung. Diese kann ein am Schaft axial fixiertes hinteres und vorderes End- oder Klemmstück sein, an dem gleitfähig je nach Vorschubrichtung der hintere oder vordere Stülpbereich des Stülpschlauchs fest anliegt, so dass der Stülpschlauch über das hintere oder vordere Endstück eine Bremskraft entgegen der gerade vorherrschenden Vorschubkraft auf den Schaft aufbringt. Alternativ hierzu kann die Synchronisationsvorrichtung ein auf den hinteren Endabschnitt des Schafts einwirkender Rollen- oder Spindelantrieb sein, der mit dem Stülpschlauchantrieb derart synchronisiert ist, dass die Vorschubgeschwindigkeit des Schafts die Hälfte der Vorschubgeschwindigkeit des inneren Schlauchabschnitts beträgt.

Der wesentliche Vorteil der aus der DE 42 42 291 A1 bekannten Endoskopievorrichtung besteht darin, dass der Enoskopschaft über dessen Gesamtlänge mit Ausnahme des vorderen, beweglichen distalen Endabschnitts von der Antriebseinrichtung, nämlich dem Doppelstülpschlauchsystem ummantelt ist, und damit nicht mit der Hohlraumwandung unmittelbar in Kontakt kommt. Darüber hinaus schafft das Doppelstülpschlauchsystem eine Art Selbstantrieb, wodurch keine Vorschubkräfte auf den Endoskopschaft von dessen Bedienungsende aus mehr aufgebracht werden müssen.

Die DE 42 42 291 A1 sowie die darin angegebene technische Lehre schafft somit die Voraussetzung zur Ausbildung neuartiger Endoskopschäfte, wie sie nunmehr Gegenstand dieser Erfindung sind.

Es hat sich gezeigt, dass der erfindungsgemäße Aufbau eines Endoskopschafts besonders kostengünstig herstellbar ist, da keine Rücksicht auf Biegesteifigkeiten in Längsrichtung des Schafts genommen werden muss, wenn ein solcher Schaft beispielsweise mit einer Antriebseinrichtung in Form eines Doppelstülpschlauchssystem bekannter Bauart eingesetzt wird. Extrudierte Kunststoffstrangprofile sind äußerst kostengünstig, wobei die Silikonhülle auf einfache Weise um den extrudierten Schlauchkörper gegossen werden kann.

Dieser Aufbau ist derart kostengünstig zu fertigen, dass der erfindungsgemäße Endoskopschaft als Einwegartikel verwendet werden kann, bei dem Sterilisationsmaßnahmen überflüssig sind.

Darüber hinaus wirkt die Silikonhülle wie ein weiches Kissen, welches den Schlauchkörper bei einem Aufprall gegen Beschädigung schützt. Es hat sich dabei ferner gezeigt, dass die Silikonhülle trotz ihrer hoch elastischen, weichen Eigenschaft als Widerlager für eine aus dem Stand der Technik bekannte Antriebseinrichtung, beispielsweise das vorstehend beschriebene Stülpschlauchantriebssystms geeignet ist.

Weitere vorteilhafte Ausgestaltungen der Erfindung sind dabei Gegenstand der übrigen Unteransprüche.

Die Erfindung wird nachstehend anhand eines bevorzugten Ausführungsbeispiels unter Bezugnahme auf die begleitenden Zeichnungen näher erläutert.

Figur 1 zeigt eine Prinzipdarstellung eines distalen Endabschnitts eines Endoskopschafts gemäß einem bevorzugten Ausführungsbeispiel der Erfindung in Querschnittsdarstellung.

Figur 2 zeigt den distalen Endabschnitt in Draufsicht als Prinzipdarstellung,

Figur 3a bis 3d zeigen den Aufbau eines Schwellkörpers,

Figur 4 zeigt die Aneinanderreihung einer Mehrzahl von Schwellkörpern in Perspektivenansicht unter Ausbildung des distalen Endes gemäß dem bevorzugten Ausführungsbeispiel und

Fig. 5 zeigt die Draufsicht eines Endoskopschafts gemäß dem bevorzugten Ausführungsbeispiel der Erfindung.

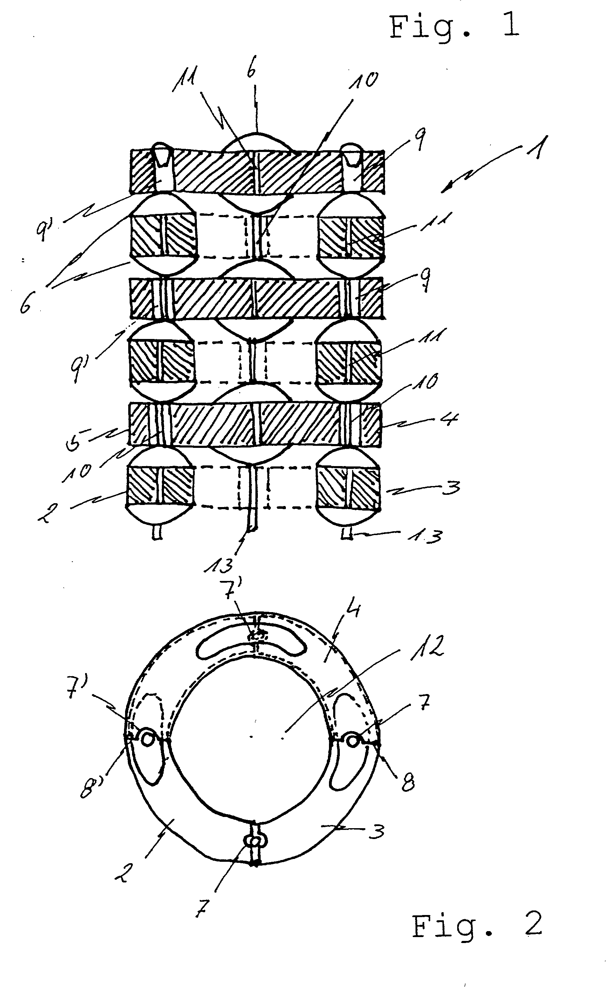

In Figur 1 ist der bewegliche distale Endabschnitt eines Endoskopschafts gemäß dem bevorzugten Ausführungsbeispiel der Erfindung als Prinzipskizze dargestellt.

Wie aus der Figur 1 zu entnehmen ist, besteht der bewegliche distale Endabschnitt 1 des erfindungsgemäßen Endoskopschafts aus einer Mehrzahl von längs neben- bzw. übereinander geschichteter Körper 2, 3, 4, wobei jede Schicht aus jeweils zwei diametrisch zueinander liegender Körpern aufgebaut ist.

In Figur 2 ist die Draufsicht des beweglichen distalen Endabschnitts 1 als Prinzipdarstellung abgebildet. Jeder Körper 2, 3, 4 besteht demzufolge aus einem halbkreisförmigen, scheibenartigen Ringelement 5, das in Umfangsrichtung gesehen in seinem Mittenabschnitt Schwellkörper 6 ausbildet. An den zwei jeweiligen Endseiten der halbkreisförmigen Ringscheibe 5 sind in Dickenrichtung der Scheibe 5 sich erstreckende Einkerbungen7, 7' vorzugsweise halbkreisförmig ausgebildet. Die halbkreisförmigen Ringscheiben 5 sind dabei in jeder Schicht an ihren beiden Endseiten 8, 8' aneinander gelegt, derart, dass die hierbei sich gegenüberliegenden Einkerbungen 7,7; 7', 7' an den jeweiligen Endseiten zwei diametrisch sich gegenüberliegende Durchgangsöffnungen 9, 9' definieren.

Wie ferner aus der Figur 1 zu entnehmen ist, sind die Körperpaare, welche unmittelbar in Längsrichtung des distalen Endabschnitts benachbart liegen, zusätzlich 90° zueinander phasenverschoben. Hierdurch ordnen sich jeweils abwechselnd die Schwellkörper 6 bezüglich der einen Schicht auf drei und neun Uhr und auf der jeweils benachbarten Schicht auf null und sechs Uhr an.

Die Schwellkörper 6 sind gemäß dem vorliegenden Ausführungsbeispiel als dehnbare Balge ausgebildet, welche pneumatisch oder hydraulisch betätigbar sind. Alternativ hierzu ist es natürlich auch möglich die Schwellkörper 6 als piezoelektrische Elemente auszubilden.

Die Schwellkörper 6, vorliegend die aufweitbaren Balge bilden einen Schwenk- bzw. Abkrümmmechanismus des beweglichen distalen Endes. Hierfür sind sämtliche Balge mit gleicher Winkelposition, d.h. die Balge auf Null-Uhr-Position, auf Drei-Uhr-Position, auf Sechs-Uhr-Position und auf Neun-Uhr-Position jeweils miteinander gekoppelt. Diese Kopplung besteht aus einem Leitungsstück 10, welches zwei längsbeabstandete Balge der gleichen Winkelposition sowohl mechanisch als auch hydraulisch bzw. pneumatisch miteinander verbindet, wobei diese Leitungsstücke 10 durch die Durchgangsöffnungen 9, 9'des dazwischen gelagerten Körperpaars jeweils verläuft. Durch die Leitungsstücke 10 wird demzufolge eine hydraulische bzw. pneumatische Fluidverbindung wie auch eine mechanische Kopplung für das Verhindern eines Auseinanderfallens der einzelnen Schichten erzeugt.

Die Balge befinden sich gemäß der Figur 1 prinzipiell an den sich gegenüberliegenden flachen Seiten der halbkreisförmigen Ringscheiben 5 und sind durch Durchgangsbohrungen 11, welche durch die Ringscheiben 5 sich erstrecken, miteinander fluidverbunden.

Wie aus der Figur 2 zu erkennen ist, wird durch die erfindungsgemäße Aneinanderlagerung der halbkreisförmigen Ringscheiben 5 pro Schicht eine zentrale Durchgangsöffnung 12 gebildet, welche sich längs des gesamten beweglichen distalen Endabschnitts 1 erstreckt und einen Arbeitskanal für das Einführen von chirurgischen Instrumenten, Hilfsinstrumenten oder Optiken bildet.

Das Funktionsprinzip des erfindungsgemäßen beweglichen distalen Endabschnitts lässt sich wie folgt zusammenfassen:

Wird in die fluid gekoppelten Balge auf einer ausgewählten Winkelposition ein Druckmedium beispielsweise eine Hydraulikflüssigkeit über die Leitungsstücke 10 eingepumpt, bewirkt dies ein Aufweiten der Balge im wesentlichen in Längsrichtung des distalen Endabschnitts 1, wodurch sich die halbkreisförmigen Ringscheiben 5 im Bereich dieser Winkelposition voneinander beabstanden. Da sämtliche weiteren Balge auf den jeweils anderen Winkelpositionen nicht druckbeaufschlagt und/oder sogar druckentspannt werden, bewirkt dies ein Abkippen jeder aus zwei Ringscheiben 5 bestehenden Schicht, wodurch sich der distale Endabschnitt über dessen gesamte Längserstreckung allmählich krümmt. Je mehr Hydraulikfluid in die gerade druckbeaufschlagten Balge eingepresst wird, desto größer wird der Krümmungsgrad des Endabschnitts, soweit, dass eine nahezu 160°-Abkrümmung erreicht werden kann.

Eine derartige Abkrümmbewegung in eine Bewegungsrichtung lässt sich natürlich überlagern durch Druckbeaufschlagung von Balgen in einer anderen Winkelposition, beispielsweise in einer hierzu 90° versetzten Winkelposition, wodurch sich eine Art Taummelbewegung des distalen Endabschnitts ergibt. Es ist auch möglich, sämtliche Balge auf allen Winkelpositionen Druck zu beaufschlagen oder zu entspannen um den distalen Endabschnitt im Bereich der Gesamtaufweitungsmöglichkeit aller längs beabstandeten Balge längs zu verschieben oder zusammenzuziehen.

Sobald das distale Ende des beweglichen Endabschnitts eine bestimmte Krümmungsposition eingenommen hat, wird die Druckbeaufschlagung der jeweilige Balge einer oder mehrerer Winkelpositionen gestoppt, wodurch aufgrund der inkompressiblen Eigenschaft des Hydraulikfluids der distale Endabschnitt in dieser Krümmungsposition fix gehalten wird.

Diese fixe Haltung ist dabei abhängig von der Elastizitiät in Radialrichtung der Balge selbst, wobei bezüglich der Konstruktion jeder halbkreisförmigen Ringscheibe eine gute Elastizität in Längsrichtung jedoch eine möglichst steife Ausgestaltung in Radialrichtung angestrebt wird, wie nachfolgend anhand einer konkreten Konstruktion näher beschrieben wird.

Wie aus der Figur 1 ferner zu entnehmen ist, weisen die halbkreisförmigen Ringscheiben 5 der gemäß Figur 1 untersten Schicht Anschlussstutzen 13 für das Anschließen eines Hydraulikleitungssystems auf. Dieses hydraulische Leitungssystem, welches in den vorliegenden Figuren nicht gezeigt ist, besteht im wesentlichen aus vier Kanälen, die durch den ebenfalls nicht gezeigten Endoskopschaft in darin ausgebildeten Arbeitskanälen geführt und an eine zentrale Hydraulikdruckquelle angeschlossen sind. Als Hydraulikdruckquelle eignet sich dabei vorzugsweise eine handbetätigbare Druckpumpe bestehend aus vier Einzelpumpen, die unabhängig voneinander oder gekoppelt betätigbar sind, derart, dass bei Druckbeaufschlagung der Balge einer Winkelposition der Druck in den Balgen auf der gegenüberliegenden Winkelposition entspannt wird. Durch eine derartige Wechselbeziehung zwischen Druckbeaufschlagung und Druckentspannung auf jeweils gegenüberliegenden Balgreihen lässt sich die Beweglichkeit des distalen Endabschnitts weiter erhöhen und die Positionierfähigkeit verbessern.

Letzteres erlaubt sogar die Exploration beispielsweise der Darmwand im Schließmuskelbereich von der Darmseite aus.

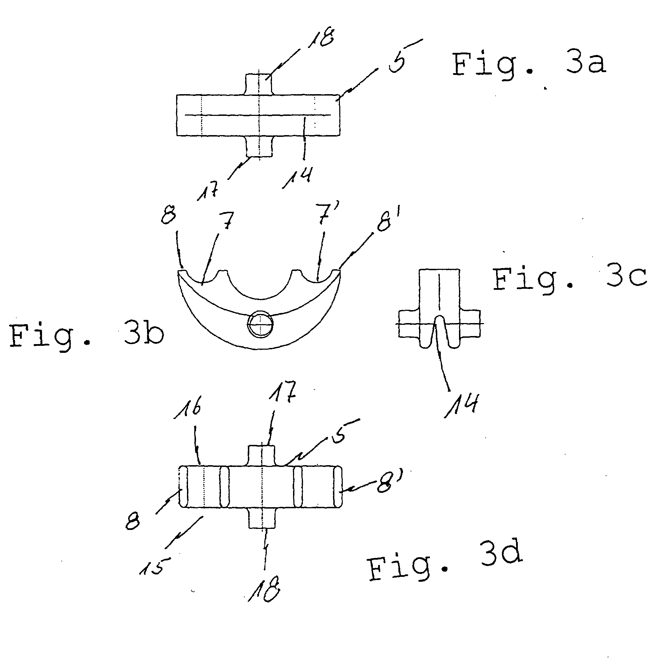

In den Figuren 3a bis 3d ist die Konstruktion einer halbkreisförmigen Ringscheibe 5 in allen Einzelheiten dargestellt. Wie hieraus zu entnehmen ist, besteht die Ringscheibe 5 aus einem Kunststoffkörper mit vorbestimmter Dicke, der einen Hohlraum ausbildet. An der radial äußeren Seite ist die Wandung des Körpers nach innen zumindest einmalig gefaltet, um einen Balg 14 auszubilden.

An den sich gegenseitig gegenüberliegenden Seitenflächen 15, 16 der Ringscheibe 5 sind Anschlussstutzen 17, 18 ausgebildet, welche fluchtend zueinander ausgerichtet sind und in den Hohlraum des Ringkörpers 5 münden. Die Anschlussstutzen 17, 18 können dabei einstückig mit der Ringscheibe 5 ausgebildet oder an diesen angeschweißt sein. Vorzugsweise besteht der Ringkörper 5, aus zwei Schalenhälften, die entlang der radial äußeren und radial inneren Seite in Umfangsrichtung miteinander verschweißt sind.

An den beiden Endflächen 8, 8' der halbkreisförmigen Ringscheibe 5 sind halbkreisförmige Einkerbungen 7, 7' in Dickenrichtung der Ringscheibe 5 ausgebildet.

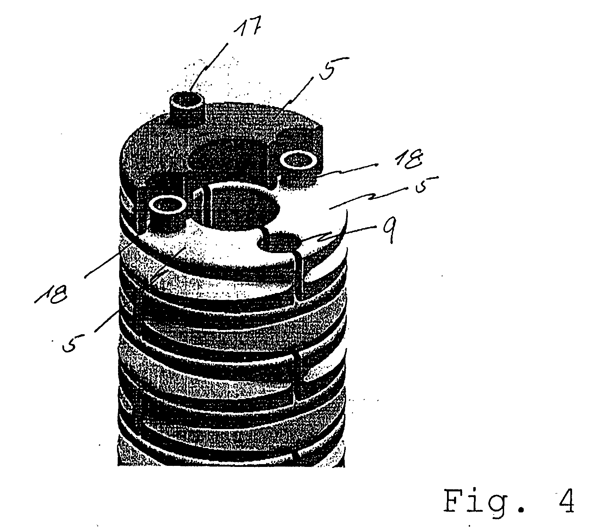

In der Figuren 4 ist der bewegliche distale Endabschnitt konstruktiv dargestellt.

Wie hieraus zu ersehen ist, bilden jeweils zwei der vorstehend beschriebenen Ringscheiben 5, welche an ihren jeweiligen Endflächen 8, 8' aneinandergelegt sind, eine Schicht, wobei die Ringscheibenpaare jeder Schicht, welche unmittelbar benachbart zueinander angeordnet sind, 90° phasenverschoben sind. Zur Fixierung der Ringscheiben 5, welche in Längsrichtung gesehen auf einer Winkelposition liegen, sind die Anschlussstutzen 17, 18, welche durch die Durchgangsöffnungen 9, 9', die durch die endseitig ausgebildeten Einkerbungen 7, 7' in der einen Schicht ausgebildet werden, hindurch sich erstrecken, miteinander verklebt oder verschweißt. Hierdurch wird das vorstehend genannte Leitungsstück 10 sowie die mechanische Verbindung der gekoppelten Ringscheiben 5 in Längsrichtung hergestellt.

Alternativ zu der vorstehend genannten Kunststoffausführung kann natürlich auch Gummi oder Gummilaminat als Material für den distalen Endabschnitt 1 verwendet werden. Der distale Endabschnitt ist dabei auf die endseitige Stirnfläche des Enoskopschafts aufgeschweißt, derart, dass die freien Anschlussstutzen 13 in den zwei untersten Schichten mit den Hydraulikkanälen längs des Hydraulikschafts in Fluidverbindung kommen.

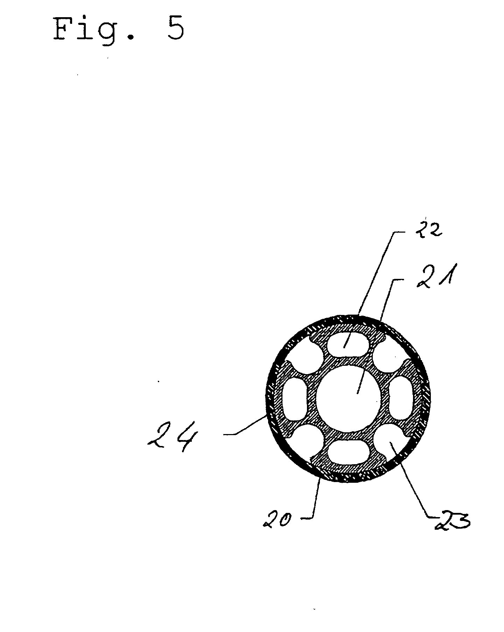

Wie in Fig. 5 dargestellt ist, hat der Endoskopschaft einen Schlauchkörper 20 aus einem estrudierten Kunststoffstrangprofil, der einen zentralen Arbeitskanal 21 mit großem Durchmesser ausbildet. Der Arbeitskanal 21 hat, wie vorstehend bereits angedeutet wurde eine durchgehende Verbindung zu der zentralen Durchgangsöffnung 12 des distalen Endabschnitts 1.

Um den Arbeitskanal 21 herum sind eine Anzahl von Funktionskanälen 22 mit kleinerem Querschnitt in dem Schlauchkörper 20 ausgebildet, die radial vom zentralen Arbeitskanal 21 beabstandet und in gleichem Winkelabstand zueinander angeordnet sind. Vorliegend sind vier Funktionskanäle 22 im Schlauchkörper 20 vorgesehen, die als Druckkanäle zur Betätigung des distalen Endabschnitts 1 mit den Anschlußstutzen in Fluidverbindung sind.

Am hinteren Endabschnitt des Endoskopschafts befindet sich die Druckmittelquelle, welche ebenfalls an die als Druckkanäle genutzten Funktionskanäle angeschlossen ist und vorzugsweise eine Einheit mit dem Endoskopschaft bildet.

Um den Schlauchkörper 20 herum ist eine Siliconhülle 24 mantelförmig angeordnet, die den Schlauchkörper 20 im wesentlichen über dessen gesamte Länge umgibt und vorzugsweise am distalen Endabschnitt 1 im Bereich von dessen Befestigungsstelle am Endoskopschaft endet oder stirnseitig mit dem distalen Endabschnitt 1 dichtend verschweißt ist.

Radial zwischen den Funktionskanälen sind kreisförmige Einkerbungen oder Nuten 23 in der Mantelfläche des Schlauchkörpers 20 ausgebildet, die sich Längs des Schlauchkörpers 20 erstrecken und in gleichen Winkelabständen zueinander angeordnet sind. Vorzugsweise liegen die Nuten 23 exakt zwischen den Funktionskanälen 22 des Schlauchkörpers 20. Die Nuten 23 sind von der Siliconhülle radial dichtend abgedeckt und bilden somit äußere Funktionskanäle, in denen Kabel beispielsweise zum Anschluß einer Kamerachips oder einer Beleuchtungseinrichtung am distalen Endabschnitt verlegt sind.

Wie eingangs bereits ausgeführt wurde, ist es besonders vorteilhaft, den Endoskopschaft als Einwegartikel auszugestalten. Um daher die Herstellungskosten zu senken, ist der distale, aus Kunststoff gefertigte Endabschnitt einfach auf das stirnseitige Ende des Endoskopschafts aufgeschweißt, wobei die Anschlußstutzen 17, 18 mit den Druckkanälen 22 des Schlauchkörpers fluidverbunden werden.

An dieser Stelle sei abschließend darauf hingewiesen, daß die Endoskopschaftkonstruktion keinesfalls nur in Kombination mit dem erfindungsgemäßen distalen Endabschnitt sondern auch mit anderen, bereits bekannten Schaftenden kombinierbar ist. Auch ist die Betätigungseinrichtung nicht auf die beschriebene Handpumpe als Einwegartikel beschränkt sondern könnte auch eine herkömmliche wiederverwendbare Druckquelle sein, an die der Endoskopschaft einfach anschließbar ist. Im letzteren Fall sind am hinteren Ende des Schafts Anschlußstellen für den Anschluß von Verbindungsleitungen zwischen Druckquelle und Funktionskanäle ausgebildet.

## Patentansprüche

1. Endoskopschaft bestehend aus einem Schlauchkörper (20), der einen zentralen Arbeitskanal (21) und eine Anzahl von Funktionskanälen (22) aufweist, sowie einem abkrümmbaren, an ein distales Ende des Schlauchkörpers angeschlossenen Endstück (1), das über die Funktionskanäle betätigbar ist, wobei der Schlauchkörper (20) aus einem extrudierten Kunststoffmaterial besteht,
**dadurch gekennzeichnet, daß**
der Schlauchkörper von einer Siliconhülle (24) umgeben ist, welche die Außenschicht des Schafts bildet, wobei
der Schlauchkörper (20) an seiner Mantelfläche eine Anzahl von in gleichem Winkelabstand angeordneten Längsausnehmungen (23) oder Nuten hat, die zusammen mit der die Mantelfläche umgreifenden Siliconhülle (24) äußere Funktionskanäle bilden.

2. Endoskopschaft nach Anspruch 1,
**dadurch gekennzeichnet, daß**
der Schlauchkörper (20) aus extrudiertem Material eine steifere Materialcharakteristik hat als die Siliconhülle (24).

3. Endoskopschaft nach Anspruch 1 oder 2,
**dadurch gekennzeichnet, daß**
die vom Schlauchkörper (20) alleine ausgebildeten Funktionskanäle (22) Druckkanäle zur Leitung eines Druckmediums wie Druckluft oder ein Hydraulikfluid sind, die auf dem gleichen Teilkreis in gleichem Winkelabstand zueinander um den zentralen Arbeitkanal (21) herum angeordnet sind und sich durch den gesamten Endoskopschaft längs erstrecken.

4. Endoskopschaft nach einem der vorstehenden Ansprüche, **gekennzeichnet durch** eine Betätigungsvorrichtung, die **durch** den Endoskopschaft hindurch mit dem abkrümmbaren Endstück für dessen Betätigung wirkverbunden ist, wobei der distale Endabschnitt aus einer Mehrzahl von längs übereinander geschichteter Schwellkörper besteht, von denen jeweils zwei diametrisch zueinander liegend eine Schicht bilden und jeweils zwei längs unmittelbar benachbarte Körperpaare 90° phasenverschoben sind.

5. Endoskopschaft nach Anspruch 4,
**dadurch gekennzeichnet, daß**
jeweils alle auf null, auf drei, auf sechs und auf neun Uhr liegenden Schwellkörper miteinander längs gekoppelt sind.

6. Endoskopschaft nach Anspruch 4,
**dadurch gekennzeichnet, daß**
die Schwellkörper als hydraulisch oder pneumatisch betätigbare Balge ausgebildet sind.

7. Endoskopschaft nach Anspruch 6,
**dadurch gekennzeichnet, daß**
die Kopplung sowohl mechanischer als auch hydraulischer oder pneumatischer Natur ist, indem zumindest ein Leitungsstück zwischen jeweils zwei miteinander gekoppelten, längs beabstandeten Balgen vorgesehen ist, welches fest an den Balgen fixiert ist.

8. Endoskopschaft nach Anspruch 7,
**dadurch gekennzeichnet, daß**
jeder Balg einen halbkreisförmig gebogenen Grundkörper hat, so daß die zwei Körperpaare jeder Schicht in Zusammenwirkung eine zentrale Durchgangsöffnung bilden.

9. Endoskopschaft nach Anspruch 8,
**dadurch gekennzeichnet, daß**
der Grundkörper einen Hohlraum bildet, und an seiner radialen Außenseite gefaltet ist.

10. Endoskopschaft nach Anspruch 9,
**dadurch gekennzeichnet, daß**
der Grundkörper an seinen beiden Stirnseiten mit Anschlußstutzen versehen ist, die in den Hohlraum münden und mit Anschlußstutzen der jeweils längs beabstandeten, in gleicher Winkelposition sich befindenden Grundkörper unter Ausbildung des Leitungsstücks verbunden sind.

11. Endoskopschaft nach Anspruch 10,
**dadurch gekennzeichnet, daß**
der Grundkörper an seinen Endflächen längs verlaufende Einkerbungen hat, die bei Bildung einer Schicht durch entsprechendes Aneinanderlegen zweier Körper zwei diametrisch gegenüberliegende Durchgangskanäle bilden, durch die jeweils ein Leitungsstück hindurch geführt ist.

## Claims

1. An endoscope shaft composed of a hose body (20) having a central working conduit (21) and a number of functional conduits (22), and of a bendable end piece (1) coupled to a distal end of the hose body, said end piece being operable by means of the functional conduits,
**characterized in that**
the hose body (20) consists of an extruded synthetic material surrounded by a silicone cover (24) which constitutes the outer layer of the shaft, wherein
the hose body (20) has, at its shell surface, a number of longitudinal recesses (23) or grooves, arranged at an equal angular distance, which form outer functional conduits together with the silicone cover (24) encompassing the shell surface.

2. An endoscope shaft according to claim 1,
**characterized in that**
the hose body (20) made of extruded material has a stiffer material characteristic than the silicone cover (24).

3. An endoscope shaft according to claim 1 or 2,
**characterized in that**
the functional conduits (22) formed by the hose body (20) are pressure conduits for guiding a compressed medium such as compressed air or a hydraulic fluid, which are arranged on the same graduated circle at an equal angular distance to each other around the central working conduit (21) and longitudinally extend through the entire endoscope shaft.

4. An endoscope shaft according to any one of the preceding claims,
**characterized by**
an operating device which is operatively connected through the endoscope shaft to the bendable end piece for the operation thereof, the distal end portion being composed of a plurality of longitudinally stacked swelling bodies two of which, respectively, are located diametrically with respect to each other to form a layer, and two longitudinally directly adjacent pairs of bodies, respectively, are phase-shifted by 90°.

5. An endoscope shaft according to claim 4,
**characterized in that**
all swelling bodies located at zero, at three, at six and at nine o'clock, respectively, are longitudinally coupled with each other.

6. An endoscope shaft according to claim 4,
**characterized in that**
the swelling bodies are formed as hydraulically or pneumatically operable bellows.

7. An endoscope shaft according to claim 6,
**characterized in that**
the coupling is both of a mechanical and hydraulic or pneumatic type **in that** at least one piece of duct is provided between two bellows, respectively, which are coupled to each other and longitudinally spaced apart, said piece of duct being fixedly mounted to the bellows.

8. An endoscope shaft according to claim 7,
**characterized in that**
each bellows has a semi-circularly bent basic body, so that the two pairs of bodies of each layer interact to form a central through hole.

9. An endoscope shaft according to claim 8,
**characterized in that**
the basic body forms a hollow and is folded at its radial outside.

10. An endoscope shaft according to claim 9,
**characterized in that**
the basic body is provided, at its two front ends, with connecting sleeves opening into the hollow and being connected to connecting sleeves of the respectively longitudinally spaced basic bodies arranged at an equal angular position while forming the piece of duct.

11. An endoscope shaft according to claim 10,
**characterized in that**
the basic body has, at its end faces, longitudinally extending notches which, upon the formation of a layer by appropriately juxtaposing two bodies, form two diametrically opposed through conduits through each of which a piece of duct is guided.

## Revendications

1. Tige endoscopique constituée par un corps de tuyau (20) présentant un canal de travail central (21) et un certain nombre de canaux de fonction (22), ainsi qu'un embout incurvable (1) relié à une extrémité distale du corps de tuyau, qui peut être actionné au moyen des canaux de fonction, le corps de tuyau (20) étant réalisé en un matériau synthétique extrudé, **caractérisée en ce que** le corps de tuyau est enrobé dans une enveloppe de silicone (24) qui forme l'aspect extérieur de la tige, le corps de tuyau (20) ayant sur sa surface enveloppe un certain nombre d'évidements longitudinaux (23) disposés avec un même intervalle angulaire, qui forment, avec l'enveloppe de silicone entourant la surface enveloppe, des canaux de fonction extérieurs.

2. Tige endoscopique selon la revendication 1, **caractérisée en ce que** le corps de tuyau (20) en matériau extrudé a une caractéristique plus raide que l'enveloppe en silicone (24).

3. Tige endoscopique selon la revendication 1 ou 2, **caractérisée en ce que** les canaux de fonction (22) formés par le corps de tuyau (20) seul sont des canaux de pression pour conduire un fluide de pression comme de l'air comprimé ou un fluide hydraulique, qui sont disposés sur le même cercle directeur avec un même intervalle angulaire entre eux autour du canal de travail central (21) et s'étendent sur toute la longueur de la tige endoscopique.

4. Tige endoscopique selon l'une des revendications précédentes, **caractérisée par** un dispositif d'actionnement qui est en liaison active, à travers la tige endoscopique, avec l'embout incurvable afin de l'actionner, la section terminale distale étant constituée d'une pluralité de corps gonflables empilés les uns sur les autres longitudinalement, dont chaque fois deux, diamétralement opposés, forment une couche et chaque fois deux paires de corps, immédiatement voisins sur la longueur, sont déphasés de 90°.

5. Tige endoscopique selon la revendication 4, **caractérisée en ce que** chaque fois tous les corps gonflables situés à zéro, trois, six et neuf heures sont couplés entre eux longitudinalement.

6. Tige endoscopique selon la revendication 4, **caractérisée en ce que** les corps gonflables sont réalisés en tant que soufflets pneumatiquement ou hydrauliquement actionnables.

7. Tige endoscopique selon la revendication 6, **caractérisée en ce que** l'accouplement est de nature aussi bien mécanique qu'hydraulique ou pneumatique, **en ce qu'**au moins une portion de conduite est prévue entre chaque fois deux soufflets reliés entre eux et espacés longitudinalement, qui est fixée rigidement sur les soufflets.

8. Tige endoscopique selon la revendication 7, **caractérisée en ce que** chaque soufflet a un corps de base courbé en demi-cercle, de sorte que les deux paires de corps de chaque couche forment en coopérant une ouverture de passage centrale.

9. Tige endoscopique selon la revendication 8, **caractérisée en ce que** le corps de base forme un espace creux et est plissé sur sa face radialement extérieure.

10. Tige endoscopique selon la revendication 9, **caractérisée en ce que** le corps de base est pourvu sur ses deux faces frontales d'ajutages de raccordement, qui débouchent dans l'espace creux et sont reliés à des ajutages de raccordement des corps de base qui se trouvent chaque fois, espacés longitudinalement, dans la même position angulaire en formant la portion de conduite.

11. Tige endoscopique selon la revendication 9, **caractérisée en ce que** le corps de base présente sur ses surfaces longitudinales des entailles s'étendant longitudinalement, qui réalisent, en formant une couche par disposition de deux corps l'un sur l'autre, deux canaux de passage diamétralement opposés, dans lesquels est guidée chaque fois une portion de conduite.
